(19) Europäisches Patentamt
European Patent Office
Office européen des brevets

(11) EP 1 537 822 A1

(12) # EUROPEAN PATENT APPLICATION

(43) Date of publication:
**08.06.2005 Bulletin 2005/23**

(51) Int Cl.[7]: **A61B 5/00**

(21) Application number: **04001951.5**

(22) Date of filing: **29.01.2004**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **03.12.2003 JP 2003404677**

(71) Applicant: **Hitachi, Ltd.**
**Chiyoda-ku, Tokyo 101-8010 (JP)**

(72) Inventors:
- **Cho, Oy-Kyung**
  **58239 Schwerte (DE)**
- **Kim, Yoon-Ok**
  **58239 Schwerte (DE)**

(74) Representative: **Strehl Schübel-Hopf & Partner**
**Maximilianstrasse 54**
**80538 München (DE)**

(54) **Blood sugar level measuring apparatus**

(57) Body-surface temperatures are measured in order to obtain information about the dissipation of heat from a body surface and information about blood flow volume. Certain parameters are calculated from the measured temperatures, blood flow volume, and constants related to hemoglobin concentration and hemoglobin oxygen saturation. The parameters are applied to a relationship between predetermined parameters and blood sugar levels to obtain a blood sugar level.

FIG. 5

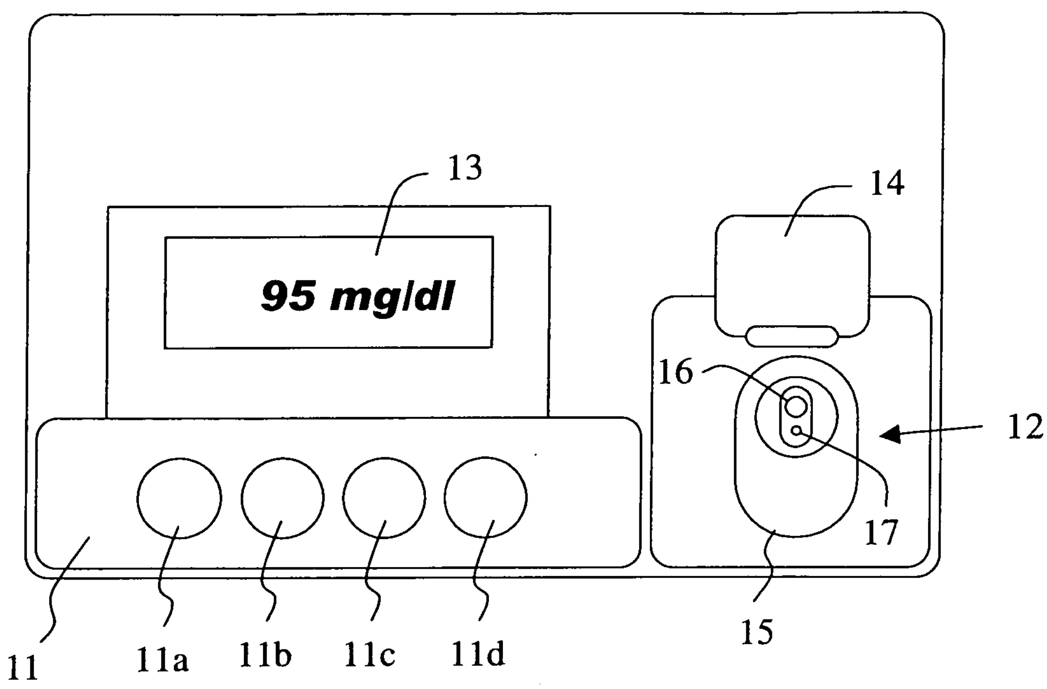


EP 1 537 822 A1

Printed by Jouve, 75001 PARIS (FR)

## Description

BACKGROUND OF THE INVENTION

Field of the Invention

The present invention relates to a non-invasive blood sugar level measuring method and apparatus for measuring glucose concentration in a living body without blood sampling.

Background Art

Hilson *et al.* report facial and sublingual temperature changes in diabetics following intravenous glucose injection (Non-Patent Document 1). Scott *et al.* discuss the issue of diabetics and thermoregulation (Non-Patent Document 2). Based on such researches, Cho *et al.* suggests a method and apparatus for determining blood glucose concentration by temperature measurement without requiring the collection of a blood sample (Patent Documents 1 and 2).

Various other attempts have been made to determine glucose concentration without blood sampling. For example, a method has been suggested (Patent Document 3) whereby a measurement site is irradiated with near-infrared light of three wavelengths, and the intensity of transmitted light as well as the temperature of the living body is detected. Then, a representative value of the second-order differentiated values of absorbance is calculated, and the representative value is corrected in accordance with the difference between the living body temperature and a predetermined reference temperature. A blood sugar level corresponding to the thus corrected representative value is then determined. An apparatus is also provided (Patent Document 4) whereby a measurement site is heated or cooled while monitoring the living body temperature. The degree of attenuation of light based on light irradiation is measured at the moment of temperature change so that the glucose concentration responsible for the temperature-dependency of the degree of light attenuation can be measured. Further, an apparatus is reported (Patent Document 5) whereby an output ratio between reference light and the light transmitted by an irradiated sample is taken, and then a glucose concentration is calculated by a linear expression of the logarithm of the output ratio and the living body temperature.

(Non-Patent Document 1)

R.M. Hilson and T.D.R. Hockaday, "Facial and sublingual temperature changes following intravenous glucose injection in diabetics," Diabete & Metabolisme, 8, pp.15-19: 1982

(Non-Patent Document 2)

A.R. Scott, T. Bennett, I.A. MacDonald, "Diabetes mellitus and thermoregulation," Can. J. Physiol. Pharmacol., 65, pp. 1365-1376: 1987

(Patent Document 1)

U.S. Patent No. 5,924,996

(Patent Document 2)

U.S. Patent No. 5,795,305

(Patent Document 3)

JP Patent Publication (Kokai) No. 2000-258343 A

(Patent Document 4)

JP Patent Publication (Kokai) No. 10-33512 A (1998)

(Patent Document 5)

JP Patent Publication (Kokai) No. 10-108857 A (1998)

SUMMARY OF THE INVENTION

Glucose (blood sugar) in blood is used for glucose oxidation reaction in cells to produce necessary energy for the maintenance of a living body. In the basal metabolism state, in particular, most of the produced energy is converted into heat energy for the maintenance of body temperature. Thus, it can be expected that there is some relationship between blood glucose concentration and body temperature. However, as is evident from the way sicknesses cause fever, the body temperature also varies due to factors other than blood glucose concentration. While methods have been proposed to determine blood glucose concentration by temperature measurement without blood sampling, they lack sufficient accuracy.

It is the object of the invention to provide a method and apparatus for determining blood glucose concentration with high accuracy based on temperature data of a subject without blood sampling.

Blood sugar is delivered to the cells throughout the human body via the blood vessel system, particularly the capillary blood vessels. In the human body, complex metabolic pathways exist. Glucose oxidation is a reaction in which, fundamentally, blood sugar reacts with oxygen to produce water, carbon dioxide, and energy. Oxygen herein refers to the oxygen delivered to the cells via blood. The amount of oxygen supply is determined by the blood hemoglobin concentration, the hemoglobin oxygen saturation, and the volume of blood flow. On the other hand, the heat produced in the body by glucose oxidation is dissipated from the body by convection, heat radiation, conduction, and so on. On the assumption that the body temperature is determined by the balance between the amount of energy produced in the body by glucose burning, namely heat production, and heat dissipation such as mentioned above, we set up the following model:

(1) The amount of heat production and the amount of heat dissipation are considered equal.
(2) The amount of heat production is a function of the blood glucose concentration and the amount of oxygen supply.
(3) The amount of oxygen supply is determined by the volume of blood flow in the capillary blood vessels.
(4) The amount of heat dissipation is mainly determined by heat convection and heat radiation.

The inventors have achieved the present invention after realizing that blood sugar levels can be accurately determined on the basis of the results of measuring the temperature of the body surface and a parameter relating to blood flow volume, in accordance with the aforementioned model. The parameters can be measured from a part of the human body, such as the fingertip. Parameters relating to convection and radiation can be determined by carrying out thermal measurements on the fingertip. The parameter relating to the volume of blood flow can be determined by measuring the amount of heat transfer from the skin.

In one example, the invention provides a blood sugar level measuring apparatus comprising:

a heat amount measuring portion for measuring a plurality of temperatures derived from a body surface and obtaining information used for calculating the amount of heat transferred by convection and the amount of heat transferred by radiation, both related to the dissipation of heat from said body surface;
a blood flow volume measuring portion for obtaining information about blood flow volume;
a first storage portion in which information about blood hemoglobin concentration and blood hemoglobin oxygen saturation is stored;
a second storage portion in which a relationship between parameters corresponding to said plurality of temperatures and to an oxygen supply volume and a blood sugar level is stored, said oxygen supply volume being determined from said hemoglobin concentration, said hemoglobin oxygen saturation and said blood flow volume;
an arithmetic portion for converting a plurality of measurement values inputted from said heat amount measuring portion and said blood flow volume measuring portion into said parameters individually, and calculating said blood sugar level by applying said parameters to said relationship stored in said second storage portion;
and a display portion for displaying the blood sugar level calculated by said arithmetic portion, wherein: said blood flow volume measuring portion comprises a body-surface contact portion, an indirect temperature detector for detecting the temperature at a position distanced from the body-surface contact portion, and a heat-conducting member connecting said body-surface contact portion and said indirect temperature detector. The blood flow volume measuring portion may comprise an adjacent temperature detector disposed adjacent to said body-surface contact portion. The heat-amount measuring portion may comprise an ambient temperature detector for measuring ambient temperature and a radiation temperature detector for measuring radiation heat from the body surface.

The first storage portion may store information about blood hemoglobin concentration and information about blood hemoglobin oxygen saturation separately. The first storage portion may store information about the product of blood hemoglobin concentration and hemoglobin oxygen saturation. The information about blood hemoglobin concentration and said information about blood hemoglobin oxygen saturation are those of the blood hemoglobin concentration and hemoglobin oxygen saturation, respectively, when resting.

In another embodiment, the invention provides a blood sugar level measuring apparatus comprising: a temperature measuring portion for measuring a plurality of temperatures from a body surface; a blood flow volume measuring portion for obtaining information about blood flow volume using the result of measurement by said temperature measuring portion; a first storage portion in which information about blood hemoglobin concentration and hemoglobin oxygen saturation is stored; a second storage portion in which a relationship between parameters and a blood sugar level is stored, said parameters corresponding to said plurality of temperatures and to oxygen supply volume individually, said oxygen supply volume being obtained based on the result of measurement by said blood flow volume measuring portion and information about the hemoglobin concentration and hemoglobin oxygen saturation in blood that are stored in said first storage portion; an arithmetic portion for converting measurement values inputted from said temperature measuring portion and said blood flow volume measuring portion into said parameters, and calculating a blood sugar level by applying said parameters to said relationship stored in said second storage portion; and a display portion for displaying the blood sugar level calculated by said arithmetic portion.

In yet another embodiment, the invention provides a blood sugar level measuring apparatus comprising: an ambient temperature measuring unit for measuring ambient temperature; a body-surface contact portion with which a body surface comes into contact; a radiation heat detector for measuring radiation heat from said body surface; a heat-conducting member disposed adjacent to said body-surface contact portion; an indirect temperature detector disposed adjacent to said heat-conducting member and away from said body-surface contact portion, for detecting the temperature at a position distanced from said body-surface contact portion; a first storage portion in which information about blood hemoglobin concentration and hemoglobin oxygen saturation is stored; an arithmetic portion including a converting portion for converting the outputs of said indirect temperature detector, said ambient temperature measuring unit and said radiation heat detector into a plurality of parameters, and a processing portion in which a relationship between said parameters and a blood sugar level is stored, said processing portion being adapted to calculate a blood sugar level by applying said parameters to said relationship; and a display portion for displaying the blood sugar level outputted from said arithmetic portion. The blood sugar level measuring apparatus may further comprise a plate with which an open end of said heat-conducting member adjacent to said body-surface contact portion is covered, and an adjacent temperature detector for detecting the temperature of said plate, wherein the output of said adjacent temperature detector is converted into said parameters by said converting portion. The heat conductivity of said plate is higher than that of said heat-conducting member.

In another embodiment, the invention provides a blood sugar level measuring apparatus comprising: an ambient temperature measuring unit for measuring ambient temperature; a body-surface contact portion with which a body surface comes into contact; a heat-conducting member disposed adjacent to a first area of said body-surface contact portion; an indirect temperature detector disposed adjacent to said heat-conducting member and away from said body-surface contact portion, for detecting the temperature at a point distanced from said body-surface contact portion; a tubular member disposed adjacent to a second area of said body-surface contact portion, one end of said tubular member being open; a radiation heat detector disposed adjacent to the other end of said tubular member, for measuring radiation heat from said body surface; a first storage portion in which information about blood hemoglobin concentration and hemoglobin oxygen saturation is stored; an arithmetic portion including a converting portion for converting the outputs of said indirect temperature detector, said ambient temperature measuring unit and said radiation heat detector into a plurality of parameters, and a processing portion in which a relationship between said parameters and a blood sugar level is stored in advance, said processing portion being adapted to calculate a blood sugar level by applying said parameters to said relationship; and a display portion for displaying the blood sugar level outputted from said arithmetic portion. The first and second areas may be disposed adjacent to each other.

In accordance with the invention, blood sugar levels can be determined in an non-invasive measurement with the same level of accuracy with that of the conventional invasive methods.

BRIEF DESCRIPTION OF THE DRAWINGS

Fig. 1 shows a model of the transfer of heat from a body surface to a block.

Fig. 2 shows changes in measurement values of temperatures $T_1$ and $T_2$ with time.

Fig. 3 shows an example of the measurement of a change in temperature $T_3$ with time.

Fig. 4 shows the relationship between measurement values obtained by various sensors and parameters derived therefrom.

Fig. 5 shows a top plan view of a non-invasive blood sugar level measuring apparatus according to the present invention.

Fig. 6 shows the procedure of operating the apparatus.

Fig. 7 shows the details of a measuring portion.
Fig. 8 is a conceptual chart illustrating the flow of data processing in the apparatus.
Fig. 9 is a conceptual chart illustrating the location where data is stored in the apparatus.
Fig. 10 shows a chart plotting the value of glucose concentration calculated in accordance with the invention and the value of glucose concentration measured by the enzymatic electrode method.

DESCRIPTION OF THE PREFERRED EMBODIMENTS

The invention will now be described by way of preferred embodiments thereof with reference made to the drawings.

Initially, the above-mentioned model will be described in more specific terms. Regarding the amount of heat dissipation, convective heat transfer, which is one of the main causes of heat dissipation, is related to temperature difference between the ambient (room) temperature and the body-surface temperature. The amount of heat dissipation due to radiation, another main cause of dissipation, is proportional to the fourth power of the body-surface temperature according to the Stefan-Boltzmann law. Thus, it can be seen that the amount of heat dissipation from the human body is related to the room temperature and the body-surface temperature. Another major factor related to the amount of heat production, the oxygen supply volume, is expressed as the product of hemoglobin concentration, hemoglobin oxygen saturation, and blood flow volume.

The hemoglobin concentration and hemoglobin oxygen saturation can be optically measured by a pulse oximeter or the like utilizing near-infrared ray. Although a feature for realizing this measurement principle may be incorporated, doing so would require the apparatus to be equipped with an optical measurement capability.

Accordingly, the hemoglobin concentration is considered a constant due to the fact that the hemoglobin concentration is stable in persons who do not have symptoms of anemia, bleeding or erythrocytosis, for example, that the hemoglobin concentration has normal values of 13 to 18 g/dL for males and 12 to 17 g/dL for females and the margin of variation from the normal values is distributed between 5% and 6%, and that the weight of a term relating to blood flow volume in a blood sugar level calculation formula, which will be described below, is less than those of other terms. Hemoglobin concentration values obtained by the aforementioned pulse oximeter or other optical equipment can be fed to the apparatus in order to make the term relating to hemoglobin concentration more appropriate, thereby achieving improvements in measurement accuracy. The hemoglobin oxygen saturation is also considered a constant due to the fact that the hemoglobin oxygen saturation is stable at 97% to 98% when he or she is undergoing aerial respiration at atmospheric pressure in a resting and relaxed state. Thus the hemoglobin concentration and hemoglobin oxygen saturation can be handled as constants, and the oxygen supply volume can be calculated as the product of the hemoglobin concentration constant, the hemoglobin oxygen saturation constant and the blood flow volume.

By handling the hemoglobin concentration and hemoglobin oxygen saturation as constants, the sensor assembly for measuring blood sugar level can be simplified by removing optical sensors or the like. Further, by eliminating the time for optical measurement and the processing of the optical measurement result, the procedure for measuring blood sugar level can be further accelerated.

The hemoglobin oxygen saturation takes on a stable value when resting, in particular. Thus, by handling the hemoglobin concentration and hemoglobin oxygen saturation as constants, improvements in measurement accuracy can be achieved during the measurement of blood sugar level when resting, in particular, and the procedure for measuring blood sugar level can be accelerated. The phrase "when resting" herein refers to the state of a subject who has been virtually motionless for about five minutes sitting on a chair or lying.

The rest is the blood flow volume, which can be measured by various methods. One example will be described below.

Fig. 1 shows a model for the description of the transfer of heat from the body surface to a solid block with a certain heat capacity as the block is brought into contact with the body surface for a certain time and then separated. The block is made of resin such as plastic or vinyl chloride. In the illustrated example, attention will be focused on the chronological variation of a temperature $T_1$ of a portion of the block in contact with the body surface, and the chronological variation of a temperature $T_2$ at a point on the block away from the body surface. The blood flow volume can be estimated by monitoring mainly the chronological variation of the temperature $T_2$ (at the spatially distant point on the block). The details will be described later.

Before the block comes into contact with the body surface, the temperatures $T_1$ and $T_2$ at the two points of the block are equal to the room temperature $T_r$. When a body-surface temperature $T_s$ is higher than the room temperature $T_r$, the temperature $T_1$ swiftly rises as the block comes into contact with the body surface, due to the transfer of heat from the skin, and it approaches the body-surface temperature $T_s$. On the other hand, the temperature $T_2$, which is lower than the temperature $T_1$ due to the dissipation of the heat conducted through the block from its surface, rises more gradually than the temperature $T_1$. The chronological variation of the temperatures $T_1$ and $T_2$ depends on the amount of heat transferred from the body surface to the block, which in turn depends on the blood flow volume in the capillary blood vessels under the skin. If the capillary blood vessels are regarded as a heat exchanger, the coefficient of heat transfer from the capillary blood vessels to the surrounding cell tissues is given as a function of the blood flow volume. Thus, by measuring the amount of heat transfer from the body surface to the block by monitoring the chronological variation of the temperatures $T_1$ and $T_2$, the amount of heat transmitted from the capillary blood vessels to the cell tissues can be estimated, which in turn makes it possible to estimate the blood flow volume.

Fig. 2 shows the chronological variation of the measured values of the temperature $T_1$ at the portion of the block in contact with the body surface and the temperature $T_2$ at the point on the block away from the body-surface contact position. As the block comes into contact with the body surface, the $T_1$ measured value swiftly rises, and it gradually drops as the block is brought out of contact.

Fig. 3 shows the chronological variation of the measured value of a temperature $T_3$ measured by a radiation temperature detector. As the temperature $T_3$ measured is that due to the radiation from the body surface, this sensor can more sensitively react to temperature changes than other sensors. Because radiation heat propagates as an electromagnetic wave, it can transmit temperature changes instantaneously. Thus, by disposing the radiation temperature detector near the position where the block comes into contact with the body surface in order to detect the heat radiated from the body surface, as shown in Fig. 7, to which reference will be made below, contact start time $t_{start}$ and contact end time $t_{end}$ between the block and body surface can be detected based on changes in temperature $T_3$. For example, a temperature threshold is set, as shown in Fig. 3, and the time at which the threshold is exceeded is taken as the contact start time $t_{start}$ and the time at which the temperature dropped below the threshold is taken as the contact end time $t_{end}$. The temperature threshold is set at 32°C, for example.

Then, the $T_1$ measured value between $t_{start}$ and $t_{end}$ is approximated by an S curve, such as a logistic curve. A logistic curve is expressed by the following equation:

$$T = \frac{b}{1 + c \times \exp(-a \times t)} + d$$

where T is temperature, and t is time.

The measured value can be approximated by determining factors a, b, c, and d by the non-linear least-squares method. For the resultant approximate expression, T is integrated between time $t_{start}$ and time tend to obtain a value $S_1$.

Similarly, an integrated value $S_2$ is calculated from the $T_2$ measured value. The smaller the $(S_1 - S_2)$ is, the larger the amount of transfer of heat from the finger surface to the position of $T_2$. $(S_1 - S_2)$ becomes larger with increasing finger contact time $t_{cont}$ $(=t_{end} - t_{start})$. Thus, $a_3/(t_{cont} \times (S_1 - S_2))$ is designated as a parameter $X_3$ indicating the volume of blood flow, where $a_3$ is a proportionality coefficient.

It will be seen from the above description that the measured quantities necessary for the determination of blood glucose concentration by the aforementioned model are the room temperature (ambient temperature), body surface temperature, temperature changes in the block in contact with the body surface, and the temperature due to radiation from the body surface.

Fig. 4 shows the relationships between the measured values provided by various sensors and the parameters derived therefrom. A block is brought into contact with the body surface, and chronological changes in the two kinds of temperatures $T_1$ and $T_2$ are measured by two temperature sensors provided at two locations of the block. Separately, the radiation temperature $T_3$ on the body surface and the room temperature $T_4$ are measured. The temperatures $T_1$, $T_2$, $T_3$, and $T_4$ provide parameters related to the volume of blood flow. The temperature $T_3$ provides a parameter related to the amount of heat transferred by radiation. The temperatures $T_3$ and $T_4$ provide parameters related to the amount of heat transferred by convection. The constants relating to hemoglobin concentration and hemoglobin oxygen saturation are stored as constants related to the oxygen supply volume. This is for the purpose of allowing the hemoglobin concentration to be adjusted for individual apparatus as necessary, given the fact that the hemoglobin concentration range is somewhat different between male and female and that females can have conditions such as anemia.

Hereafter, an example of the apparatus for non-invasively measuring blood sugar levels according to the principle of the invention will be described.

Fig. 5 shows a top plan view of the non-invasive blood sugar level measuring apparatus according to the invention. While in this example the skin on the ball of the fingertip is used as the body surface, other parts of the body surface may be used.

On the upper surface of the apparatus are provided an operating portion 11, a measuring portion 12 where the finger to be measured is to be placed, and a display portion 13 for displaying the result of measurement, the state of the apparatus, measured values, and so on. The operating portion 11 includes four push buttons 11a to 11d for operating the apparatus. The measuring portion 12 has a cover 14 which, when opened (as shown), reveals a finger rest portion 15 with an oval periphery. The finger rest portion 15 accommodates an opening end 16 of a radiation temperature sensor portion, and a contact temperature sensor portion 17.

Fig. 6 shows the procedure for operating the apparatus. As one of the buttons on the operating portion is depressed and the apparatus is turned on, an indication "Warming up" is displayed on the LCD portion while the electronic circuits in the apparatus are being warmed up. At the same time, a check program is activated to automatically check the electronic circuits. When the warm-up phase is over, an indication "Place your finger" appears on the LCD portion. As the user places his or her finger on the finger rest portion, the LCD portion displays a countdown. When the countdown is over, an indication "Put your finger away" appears on the LCD portion. As the user puts his or her finger away from the finger rest portion, the LCD portion indicates "Processing data." Thereafter, a blood sugar level is displayed on the LCD portion. At this point, the displayed blood sugar level is stored in an IC card, together with the date and time. The user reads the displayed blood sugar level and pushes another button on the operating portion. About one minute later, the apparatus displays a message "Place your finger" on the LCD portion, thus indicating that the apparatus is ready for the next cycle of measurement.

Fig. 7 shows the measuring portion in detail. Fig. 7(a) is a top plan view, and (b) is a cross section along line X-X of (a).

First, temperature measurement by the non-invasive blood sugar level measuring apparatus according to the invention will be described. A thin plate 21 of a highly heat-conductive material, such as gold, is disposed on a portion of where a measured portion (ball of the finger) is to come into contact. A bar-shaped heat-conductive member 22 made of material with a heat conductivity lower than that of the plate 21, such as polyvinylchloride, is thermally connected to the plate 21 and extends into the apparatus. The temperature sensors include a thermistor 23, which is a temperature detector adjacent the measured portion for measuring the temperature of the plate 21. There is also a thermistor 24, which is an indirect temperature detector for the measured portion for measuring the temperature of a portion of the heat-conducting member away from the plate 21 by a certain distance. An infrared lens 25 is disposed inside the apparatus at such a position that the measured portion (ball of the finger) placed on the finger rest portion 15 can be seen through the lens. Below the infrared lens 25 is disposed a pyroelectric detector 27 via an infrared radiation-transmitting window 26. Another thermistor 28 is disposed near the pyroelectric detector 27.

Thus, the temperature sensor portion of the measuring portion has four temperature sensors, and they measure four kinds of temperatures as follows:

(1) Temperature on the finger surface (thermistor 23): $T_1$
(2) Temperature of the heat-conducting member (thermistor 24): $T_2$
(3) Temperature of radiation from the finger (pyroelectric detector 27): $T_3$
(4) Room temperature (thermistor 28): $T_4$

Fig. 8 is a conceptual chart illustrating the flow of data processing in the apparatus. The apparatus according to the present example is equipped with four sensors, namely thermistor 23, thermistor 24, pyroelectric detector 27, and thermistor 28.

Four kinds of analog signals are supplied via individual amplifiers A1 to A4 and digitally converted by analog/digital converters AD1 to AD4. Based on the digitally converted values, parameters $x_i$ (i=1, 2, 3) are calculated. The following are specific descriptions of $x_i$ (where $a_i$ to $a_3$ are proportionality coefficients):

The parameter proportional to the blood flow volume is equivalent to the parameter relating to the oxygen supply volume. A coefficient $a_3$ of the parameter proportional to the oxygen supply volume is the product of each of hemoglobin concentration $c_1$ and hemoglobin oxygen saturation $c_2$ and a proportionality coefficient $a_3'$.

Parameter proportional to heat radiation

$$x_1 = a_1 \times (T_3)^4$$

Parameter proportional to heat convection

$$x_2 = a_2 \times (T_4 - T_3)$$

Parameter proportional to oxygen supply volume

$$x_3 = a_3 \times \left( \frac{1}{t_{CONT} \times (S_1 - S_2)} \right)$$

Then, normalized parameters are calculated from mean values and standard deviations of parameters $x_i$ obtained from actual data pertaining to large numbers of able-bodied people and diabetic patients. A normalized parameter $X_i$ (where i=1, 2, 3) is calculated from each parameter $x_i$ according to the following equation:

$$X_i = \frac{x_i - \bar{x}_i}{SD(x_i)}$$

where

$x_i$: parameter

$\bar{x}_i$: mean value of the parameter

$SD(x_i)$: standard deviation of the parameter

Calculations are conducted to convert the above three normalized parameters into glucose concentration to be eventually displayed. Fig. 9 shows a functional block diagram of the apparatus. The present apparatus is powered by a battery 41. Signals measured by a sensor portion 43 comprising temperature sensors are fed to analog/digital converters 44 (analog/digital converters AD1 to AD4) provided for individual signals, where they are converted into digital signals. Peripheral circuits to a microprocessor 45 include the analog/digital converters AD1 to AD4, an LCD 13 and a RAM 42. These peripheral circuits are individually accessed by the microprocessor 45 via a bus line 46. Push buttons 11a to 11d are connected to the microprocessor 45, which includes a ROM 47 in which software is stored. The microprocessor 45 can receive instructions from the outside by the pressing of the buttons 11 a to 11d.

The ROM 47 included in the microprocessor 45 stores a program necessary for computations, i.e., it has the function of an arithmetic unit. The microprocessor 45 further includes a hemoglobin concentration constant storage portion 48 for storing hemoglobin concentration constants, and a hemoglobin oxygen saturation constant storage portion 49 for storing hemoglobin oxygen saturation constants. After the measurement of the finger is finished, the computing program calls optimum constants from the hemoglobin concentration storage portion 48 and hemoglobin oxygen saturation constant storage portion 49 and perform calculations. A memory area necessary for computations is ensured in the RAM 42 similarly incorporated into the apparatus. The result of computations is displayed on the LCD portion.

The ROM stores, as a constituent element of the program necessary for the computations, a function for determining glucose concentration C in particular. The function is defined as follows. C is expressed by a below-indicated equation (1), where $a_i$ (i=0, 1, 2, 3) is determined from a plurality of pieces of measurement data in advance according to the following procedure:

(1) A multiple regression equation is created that indicates the relationship between the normalized parameter and the glucose concentration C.

(2) Normalized equations (simultaneous equations) relating to the normalized parameter are obtained from an equation obtained by the least-squares method.

(3) Values of coefficient $a_i$ (i=0, 1, 2, 3) are determined from the normalized equation and then substituted into the multiple regression equation.

Initially, the regression equation (1) indicating the relationship between the glucose concentration C and the normalized parameters $X_1$, $X_2$ and $X_3$ is formulated.

$$\begin{aligned} C &= f(X_1, X_2, X_3) \\ &= a_0 + a_1 X_1 + a_2 X_2 + a_3 X_3 \quad \text{......(1)} \end{aligned}$$

Then, the least-squares method is employed to obtain a multiple regression equation that would minimize the error with respect to a measured value Ci of glucose concentration according to an enzyme electrode method. When the sum of squares of the residual is D, D is expressed by the following equation (2):

$$\begin{aligned} D &= \sum_{i=1}^{n} d_i^{\,2} \\ &= \sum_{i=1}^{n} (C_i - f(X_{i1}, X_{i2}, X_{i3}))^2 \\ &= \sum_{i=1}^{n} \{C_i - (a_0 + a_1 X_{i1} + a_2 X_{i2} + a_3 X_{i3})\}^2 \quad ......(2) \end{aligned}$$

The sum of squares of the residual D becomes minimum when partial differentiation of equation (2) with respect to $a_0$ to $a_3$ gives zero. Thus, we have the following equations:

$$\begin{aligned} \frac{\partial D}{\partial a_0} &= -2\sum_{i=1}^{n} \{C_i - (a_0 + a_1 X_{i1} + a_2 X_{i2} + a_3 X_{i3})\} = 0 \\ \frac{\partial D}{\partial a_1} &= -2\sum_{i=1}^{n} X_{i1}\{C_i - (a_0 + a_1 X_{i1} + a_2 X_{i2} + a_3 X_{i3})\} = 0 \\ \frac{\partial D}{\partial a_2} &= -2\sum_{i=1}^{n} X_{i2}\{C_i - (a_0 + a_1 X_{i1} + a_2 X_{i2} + a_3 X_{i3})\} = 0 \\ \frac{\partial D}{\partial a_3} &= -2\sum_{i=1}^{n} X_{i3}\{C_i - (a_0 + a_1 X_{i1} + a_2 X_{i2} + a_3 X_{i3})\} = 0 \quad ......(3) \end{aligned}$$

When the mean values of C and $X_1$ to $X_3$ are $C_{mean}$ and $X_{1mean}$ to $X_{3mean}$, respectively, since $X_{imean}$=0 (i=1 to 3), equation (1) yields equation (4) thus:

$$\begin{aligned} a_0 &= C_{mean} - a_1 X_{1mean} - a_2 X_{2mean} - a_3 X_{3mean} \\ &= C_{mean} \qquad (4) \end{aligned}$$

The variation and covariation between the normalized parameters are expressed by equation (5). Covariation between the normalized parameter $X_i$ (i=1 to 3) and C is expressed by equation (6).

$$S_{ij} = \sum_{k=1}^{n} (X_{ki} - X_{imean})(X_{kj} - X_{jmean}) = \sum_{k=1}^{n} X_{ki} X_{kj} \quad (i, j = 1,2,3) \quad ......(5)$$

$$S_{iC} = \sum_{k=1}^{n} (X_{ki} - X_{imean})(C_k - C_{mean}) = \sum_{k=1}^{n} X_{ki}(C_k - C_{mean}) \quad (i = 1,2,3) \quad ......(6)$$

Substituting equations (4), (5), and (6) into equation (3) and rearranging yields simultaneous equations (normalized equations) (7). Solving equations (7) yields $a_1$ to $a_3$.

$$a_1S_{11} + a_2S_{12} + a_3S_{13} = S_{1C}$$
$$a_1S_{21} + a_2S_{22} + a_3S_{33} = S_{2C}$$
$$a_1S_{31} + a_2S_{32} + a_3S_{33} = S_{3C} \quad (7)$$

Constant term $a_0$ is obtained by means of equation (4). The thus obtained $a_i$ (i=0, 1, 2, 3) is stored in ROM at the time of manufacture of the apparatus. In actual measurement using the apparatus, the normalized parameters $X_1$ to $X_3$ obtained from the measured values are substituted into regression equation (1) to calculate the glucose concentration C.

Hereafter, an example of the process of calculating the glucose concentration will be described. The coefficients in equation (1) are determined in advance based on a large quantity of data obtained from able-bodied persons and diabetic patients. The ROM in the microprocessor stores the following formula for the calculation of glucose concentration:

$$C = 101.7 + 25.8 \times X_1 - 23.2 \times X_2 - 12.9 \times X_3$$

$X_1$ to $X_3$ are the results of normalization of parameters $x_1$ to $x_3$. Assuming the distribution of the parameters is normal, 95% of the normalized parameters take on values between -2 and +2.

In an example of measured values for an able-bodied person, substituting normalized parameters $X_1$=-0.06, $X_2$=+0.04 and $X_3$=+0.10 in the above equation yields C=101 mg/dL. In an example of measured values for a diabetic patient, substituting normalized parameters $X_1$=+1.35, $X_2$=-1.22 and $X_3$=-1.24 in the equation yields C=181 mg/dL. In the above equation, the hemoglobin concentration and hemoglobin oxygen saturation are rendered into constants of 15 g/dL and 97%, respectively.

Hereafter, the results of measurement by the conventional enzymatic electrode method and those by the embodiment of the invention will be described. In the enzymatic electrode method, a blood sample is reacted with a reagent and the amount of resultant electrons is measured to determine glucose concentration. When the glucose concentration was 93 mg/dL according to the enzymatic electrode method in an example of measured values for an able-bodied person, substituting normalized parameters $X_1$=-0.06, $X_2$=+0.04 and $X_3$=+0.10 obtained by measurement at the same time according to the inventive method into the above equation yielded C=101 mg/dL. Further, when the glucose concentration was 208 mg/dL according to the enzymatic electrode method in an example of measurement values for a diabetic patient, substituting $X_1$=+1.35, $X_2$=-1.22 and $X_3$=-1.24 obtained by measurement at the same time according to the inventive method yielded C=181 mg/dL. Although the calculation results indicate an error of about 13%, this level of accuracy is considered sufficient because normally errors between 15% and 20% are considered acceptable in blood sugar level measuring apparatuses in general. Thus, it has been confirmed that the method of the invention can allow glucose concentrations to be determined with high accuracy.

Fig. 10 shows a chart plotting on the vertical axis the values of glucose concentration calculated by the inventive method and on the horizontal axis the values of glucose concentration measured by the enzymatic electrode method, based on measurement values obtained from a plurality of patients. A good correlation is obtained by measuring according to the invention (correlation coefficient = 0.8932).

## Claims

**1.** A blood sugar level measuring apparatus comprising:

a heat amount measuring portion for measuring a plurality of temperatures derived from a body surface and obtaining information used for calculating the amount of heat transferred by convection and the amount of heat transferred by radiation, both related to the dissipation of heat from said body surface;
a blood flow volume-measuring portion for obtaining information about blood flow volume;
a first storage portion in which information about blood hemoglobin concentration and blood hemoglobin oxygen saturation is stored;
a second storage portion in which a relationship between parameters corresponding to said plurality of temperatures and to an oxygen supply volume and a blood sugar level is stored, said oxygen supply volume being determined from said hemoglobin concentration, said hemoglobin oxygen saturation and said blood flow volume;
an arithmetic portion for converting a plurality of measurement values inputted from said heat amount measuring portion and said blood flow volume measuring portion into said parameters individually, and calculating said blood sugar level by applying said parameters to said relationship stored in said second storage portion; and
a display portion for displaying the blood sugar level calculated by said arithmetic portion,

wherein:

said blood flow volume measuring portion comprises a body-surface contact portion, an indirect temperature detector for detecting the temperature at it position distanced from the body-surface contact portion, and a heat-conducting member connecting said body-surface contact portion and said indirect temperature detector.

**2.** The blood sugar level measuring apparatus according to claim 1, wherein said blood flow volume measuring portion comprises an adjacent temperature detector disposed adjacent to said body-surface contact portion.

**3.** The blood sugar level measuring apparatus according to claim 1, wherein said heat-amount measuring portion comprises an ambient temperature detector for measuring ambient temperature and a radiation temperature detector for measuring radiation heat from the body surface.

**4.** A blood sugar level measuring apparatus comprising:

a temperature measuring portion for measuring a plurality of temperatures from a body surface;
a blood flow volume measuring portion for obtaining information about blood flow volume using the result of measurement by said temperature measuring portion;
a first storage portion in which information about blood hemoglobin concentration and hemoglobin oxygen saturation is stored;
a second storage portion in which a relationship between parameters and a blood sugar level is stored, said parameters corresponding to said plurality of temperatures and to oxygen supply volume individually, said oxygen supply volume being obtained based on the result of measurement by said blood flow volume measuring portion and information about the hemoglobin concentration and hemoglobin oxygen saturation in blood that are stored in said first storage portion;
an arithmetic portion for converting measurement values inputted from said temperature measuring portion and said blood flow volume measuring portion into said parameters, and calculating a blood sugar level by applying said parameters to said relationship stored in said second storage portion; and
a display portion for displaying the blood sugar level calculated by said arithmetic portion.

**5.** The blood sugar level measuring apparatus according to claim 7, wherein said blood flow volume measuring portion comprises a body-surface contact portion, an adjacent temperature detector disposed adjacent to said body-surface contact portion, an indirect temperature detector for detecting the temperature at a position distanced from said body-surface contact portion, and a heat-conducting member connecting said body-surface contact portion and said indirect temperature detector.

**6.** The blood sugar level measuring apparatus of any preceding claim, wherein said first storage portion stores information about blood hemoglobin concentration and information about blood hemoglobin oxygen saturation separately.

**7.** The blood sugar level measuring apparatus of any preceding claim, wherein said first storage portion stores information about the product of blood hemoglobin concentration and hemoglobin oxygen saturation.

**8.** A blood sugar level measuring apparatus comprising:

an ambient temperature measuring unit for measuring ambient temperature;
a body-surface contact portion with which a body surface comes into contact;
a radiation heat detector for measuring radiation heat from said body surface;
a heat-conducting member disposed adjacent to said body-surface contact portion;

an indirect temperature detector disposed adjacent to said heat-conducting member and away from said body-surface contact portion, for detecting the temperature at a position distanced from said body-surface contact portion;
a first storage portion in which information about blood hemoglobin concentration and hemoglobin oxygen saturation is stored;
an arithmetic portion including a converting portion for converting the outputs of said indirect temperature detector, said ambient temperature measuring unit and said radiation heat detector into a plurality of parameters, and a processing portion in which a relationship between said parameters and a blood sugar level is stored, said processing portion being adapted to calculate a blood sugar level by applying said parameters to said relationship; and
a display portion for displaying the blood sugar level outputted from said arithmetic portion.

**9.** The blood sugar level measuring apparatus according to claim 12, further comprising a plate with which an open end of said heat-conducting member adjacent to said body-surface contact portion is covered, and an adjacent temperature detector for detecting the temperature of said plate, wherein the output of said adjacent temperature detector is converted into said parameters by said converting portion.

**10.** The blood sugar level measuring apparatus according to claim 13, wherein the heat conductivity of said plate is higher than that of said heat-conducting member.

**11.** A blood sugar level measuring apparatus comprising:

an ambient temperature measuring unit for measuring ambient temperature;
a body-surface contact portion with which a body surface comes into contact;
a heat-conducting member disposed adjacent to a first area of said body-surface contact portion;
an indirect temperature detector disposed adjacent to said heat-conducting member and away from said body-surface contact portion, for detecting the temperature at a point distanced from said body-surface contact portion;
a tubular member disposed adjacent to a second area of said body-surface contact portion, one end of said tubular member being open;
a radiation heat detector disposed adjacent to the other end of said tubular member, for measuring radiation heat from said body surface;
a first storage portion in which information about blood hemoglobin concentration and hemoglobin oxygen saturation is stored;
an arithmetic portion including a converting portion for converting the outputs of said indirect temperature detector, said ambient temperature measuring unit and said radiation heat detector into a plurality of parameters, and a processing portion in which a relationship between said parameters and a blood sugar level is stored in advance, said processing portion being adapted to calculate a blood sugar level by applying said parameters to said relationship; and
a display portion for displaying the blood sugar level outputted from said arithmetic portion.

**12.** The blood sugar level measuring apparatus according to claim 16, wherein said first and second areas are disposed adjacent to each other.

**13.** The blood sugar level measuring apparatus of any preceding claim, wherein said information about blood hemoglobin concentration and said information about blood hemoglobin oxygen saturation are those of the blood hemoglobin concentration and hemoglobin oxygen saturation, respectively, when resting.

FIG. 1

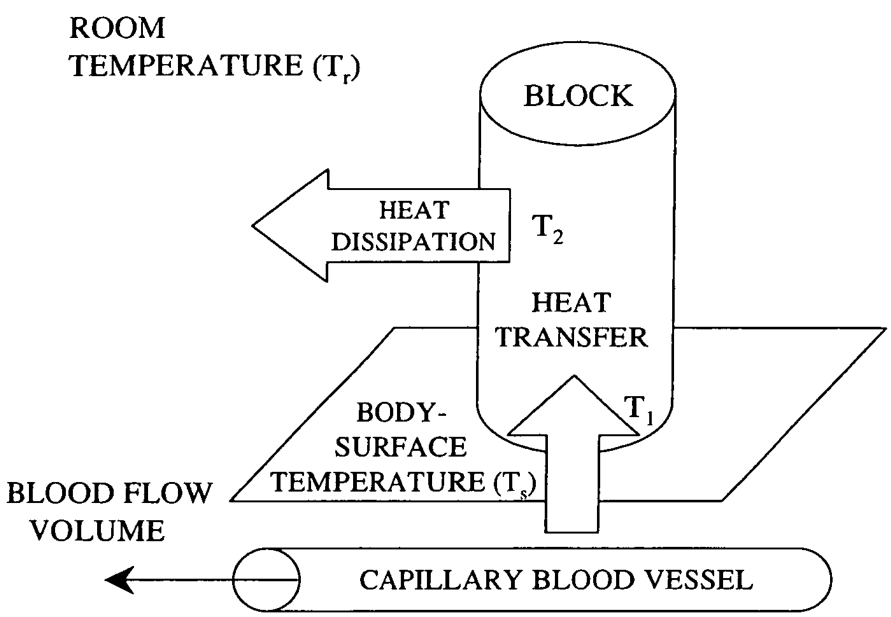
ROOM
TEMPERATURE ($T_r$)
BLOCK
HEAT
DISSIPATION
$T_2$
HEAT
TRANSFER
BODY-
SURFACE
TEMPERATURE ($T_s$)
$T_1$
BLOOD FLOW
VOLUME
CAPILLARY BLOOD VESSEL

FIG. 2

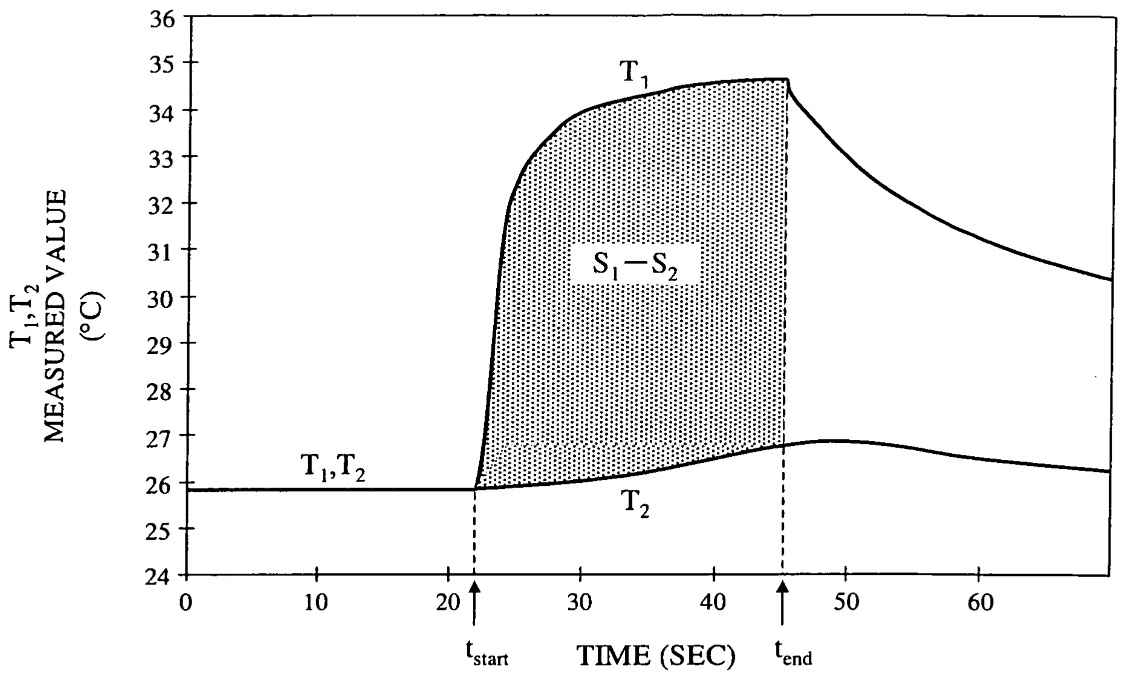
T1,T2
MEASURED VALUE
(°C)
36
35
34
33
32
31
30
29
28
27
26
25
24
T1
S1−S2
T1,T2
T2
0
10
20
30
40
50
60
tstart
TIME (SEC)
tend

FIG. 3

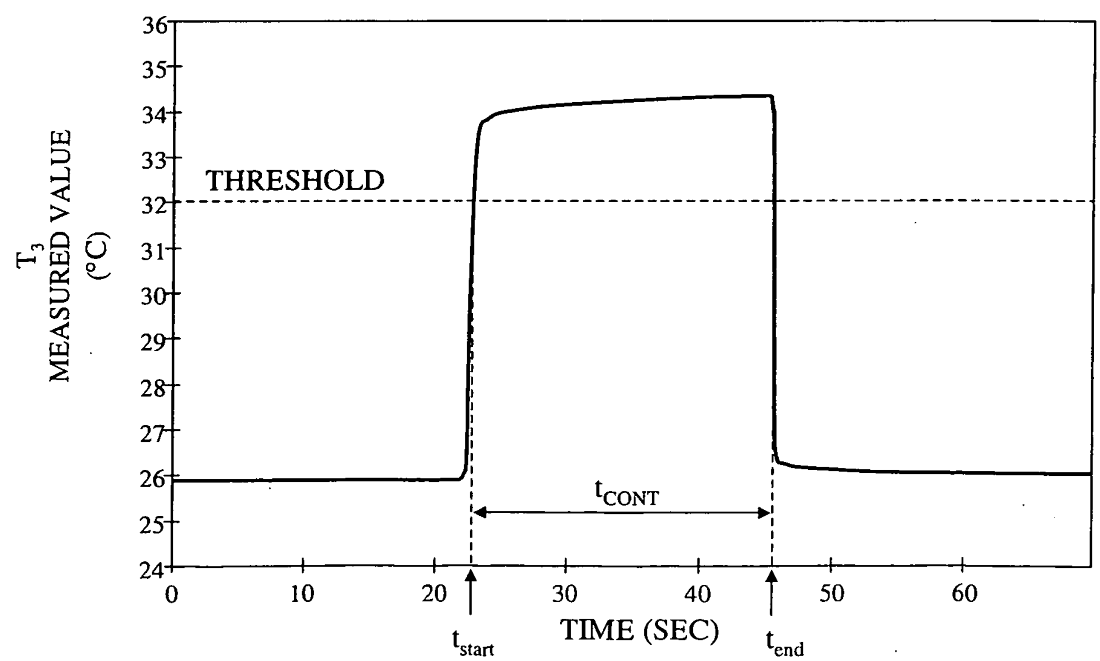
T3
MEASURED VALUE
(°C)
36
35
34
33
32
31
30
29
28
27
26
25
24
THRESHOLD
tCONT
0
10
20
30
40
50
60
tstart
TIME (SEC)
tend

FIG. 4

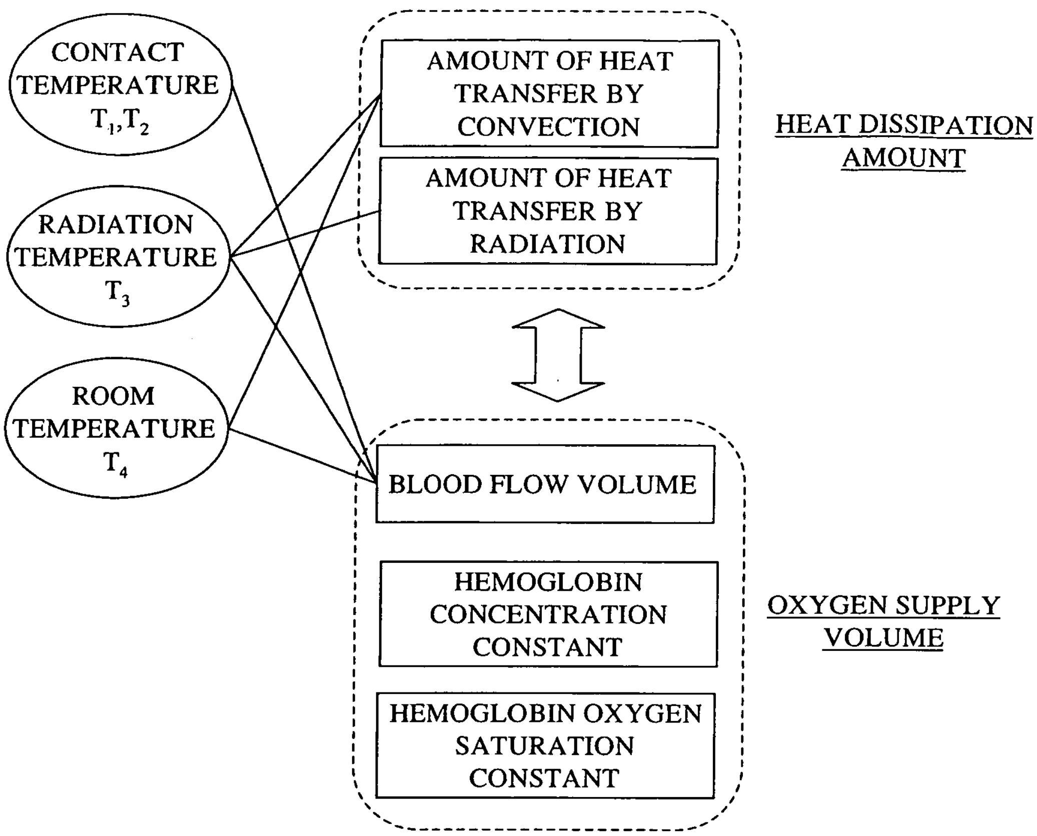
CONTACT TEMPERATURE $T_1,T_2$
RADIATION TEMPERATURE $T_3$
ROOM TEMPERATURE $T_4$
AMOUNT OF HEAT TRANSFER BY CONVECTION
AMOUNT OF HEAT TRANSFER BY RADIATION
HEAT DISSIPATION AMOUNT
BLOOD FLOW VOLUME
HEMOGLOBIN CONCENTRATION CONSTANT
HEMOGLOBIN OXYGEN SATURATION CONSTANT
OXYGEN SUPPLY VOLUME

FIG. 5

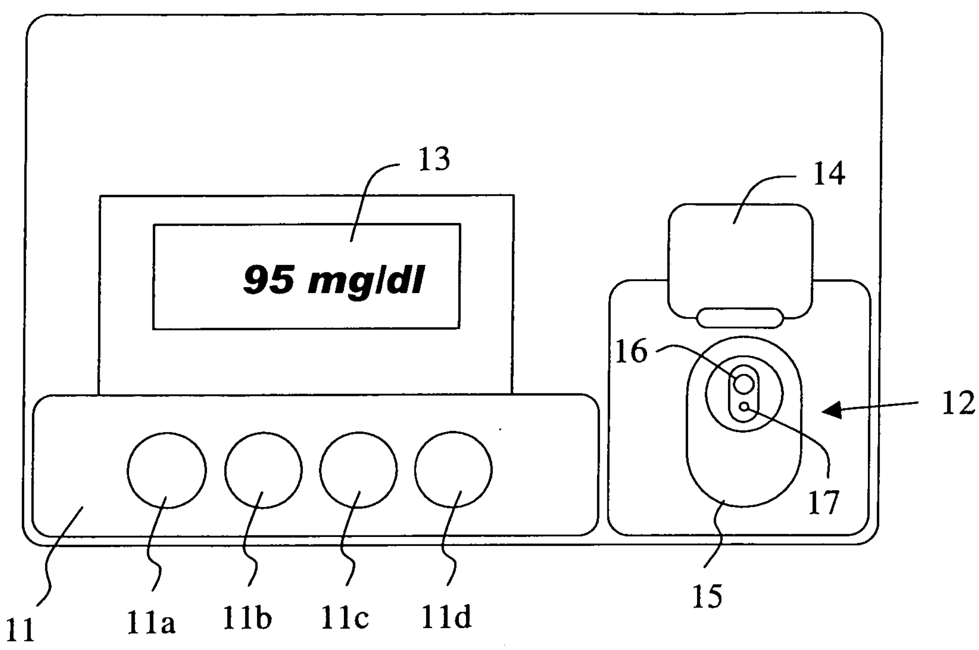

FIG. 6

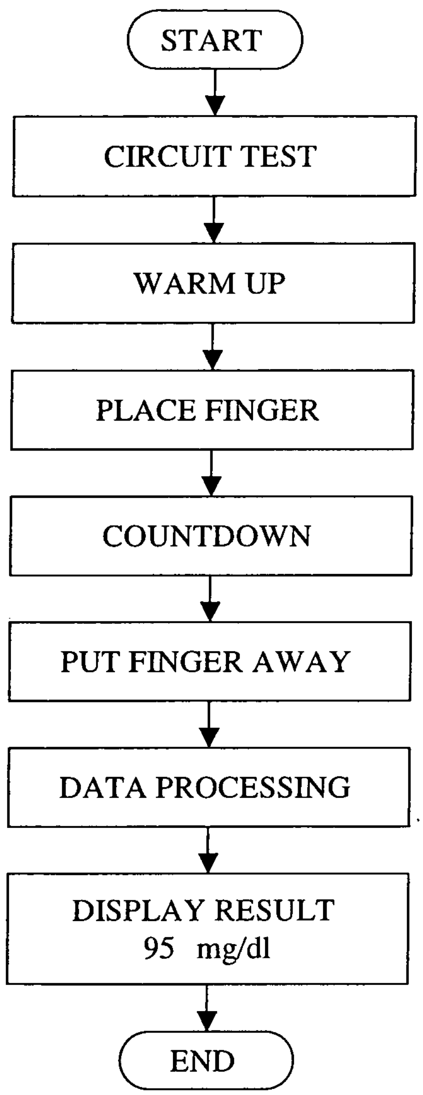
START
CIRCUIT TEST
WARM UP
PLACE FINGER
COUNTDOWN
PUT FINGER AWAY
DATA PROCESSING
DISPLAY RESULT
95 mg/dl
END

FIG. 7A

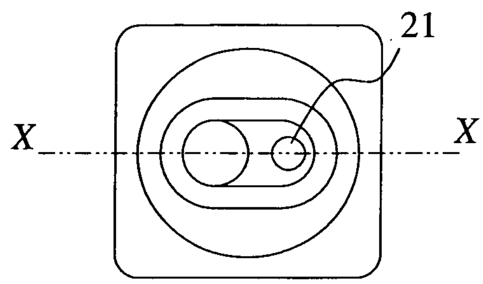


FIG. 7B

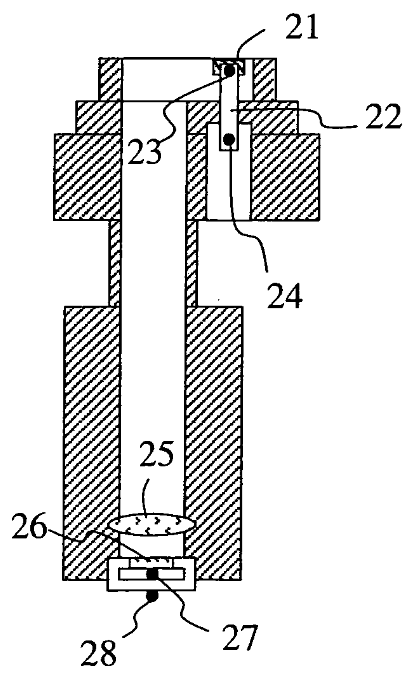

FIG. 8

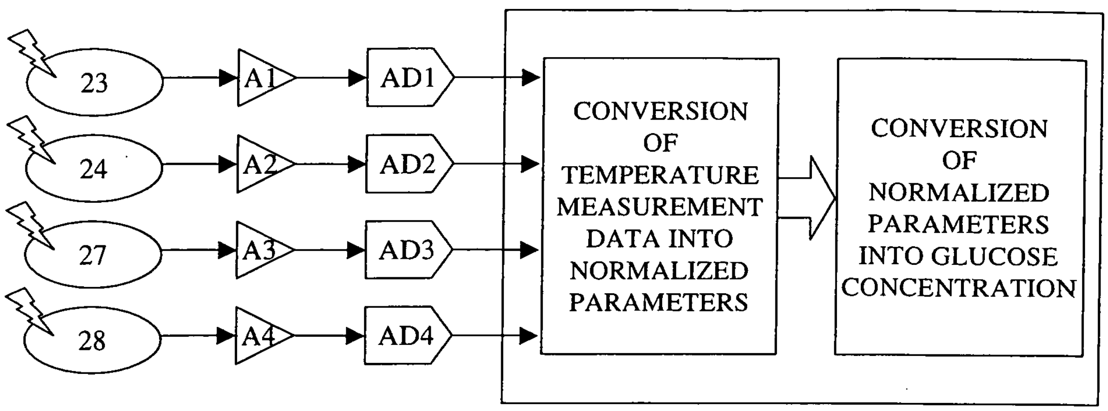
23
24
27
28
A1
A2
A3
A4
AD1
AD2
AD3
AD4
CONVERSION OF TEMPERATURE MEASUREMENT DATA INTO NORMALIZED PARAMETERS
CONVERSION OF NORMALIZED PARAMETERS INTO GLUCOSE CONCENTRATION

FIG. 9

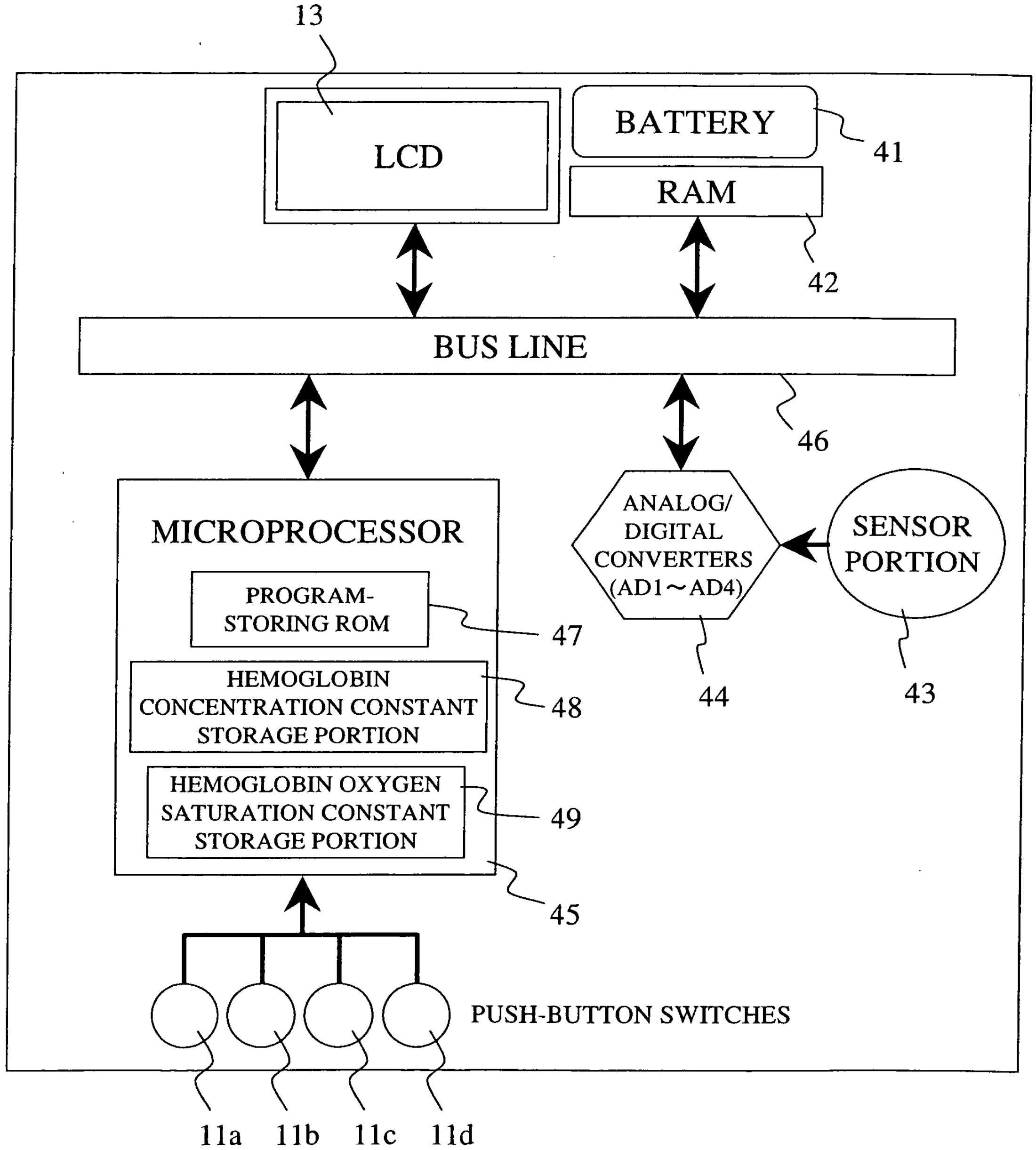
13
LCD
BATTERY
41
RAM
42
BUS LINE
46
MICROPROCESSOR
PROGRAM-
STORING ROM
47
HEMOGLOBIN
CONCENTRATION CONSTANT
STORAGE PORTION
48
HEMOGLOBIN OXYGEN
SATURATION CONSTANT
STORAGE PORTION
49
45
ANALOG/
DIGITAL
CONVERTERS
(AD1～AD4)
44
SENSOR
PORTION
43
PUSH-BUTTON SWITCHES
11a
11b
11c
11d

FIG.10

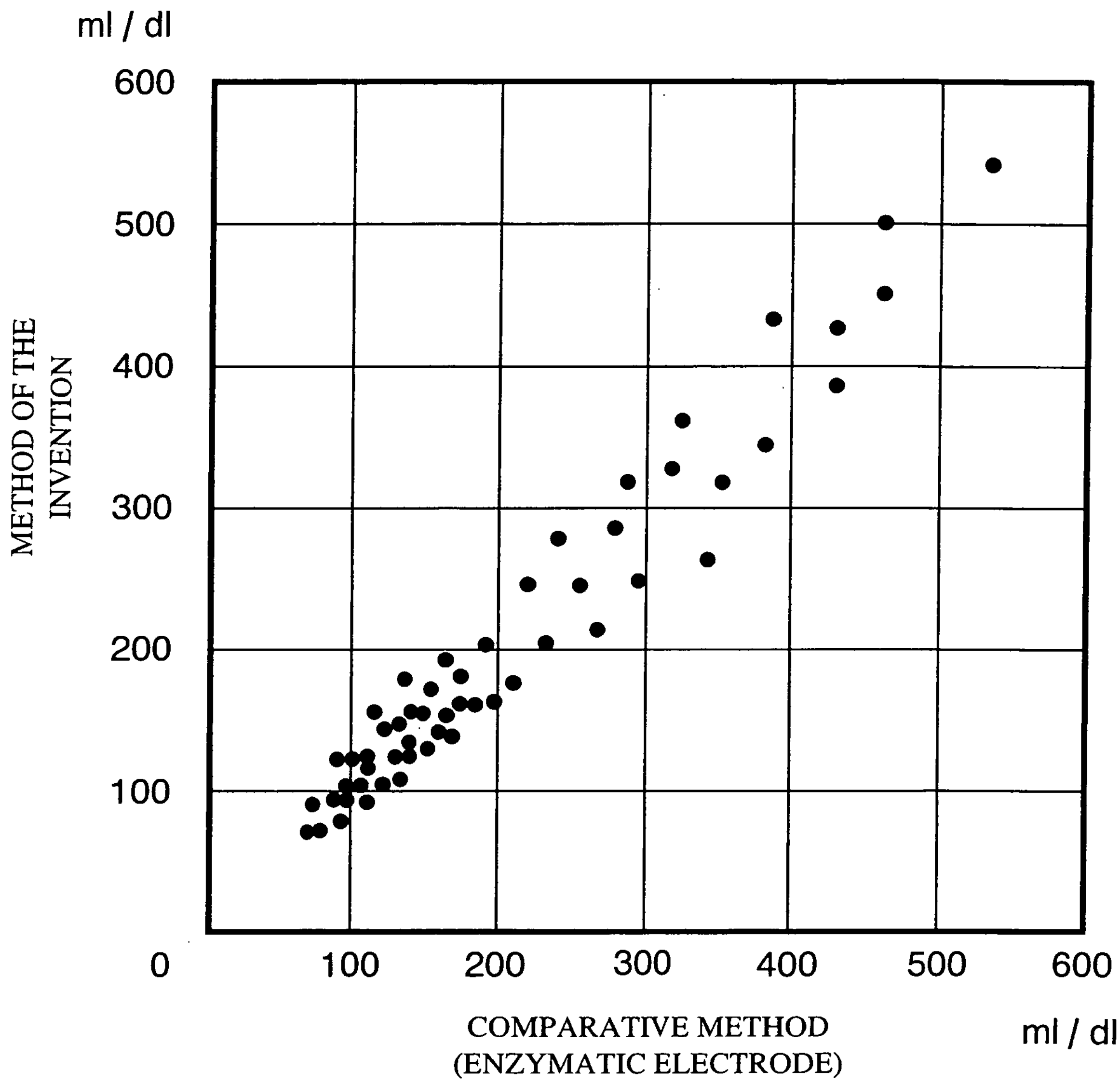
ml / dl
600
500
400
300
200
100
0
METHOD OF THE INVENTION
100
200
300
400
500
600
COMPARATIVE METHOD (ENZYMATIC ELECTRODE)
ml / dl

European Patent Office

# EUROPEAN SEARCH REPORT

Application Number
EP 04 00 1951

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,Y | US 5 924 996 A (CHO ET AL) 20 July 1999 (1999-07-20) * column 6, line 31 - column 7, line 5; figures 1-3 * * column 7, line 32 - column 8, line 3 * * claims 4,8,9,11,19 * ----- | 1-13 | A61B5/00 |
| Y | WO 01/28414 A (KAUFMANN-KIM, YUN-OAK; CHO, OK-KYUNG) 26 April 2001 (2001-04-26) * page 3, line 19 - page 5, line 2 * ----- | 1-13 | |
| E | EP 1 484 006 A (HITACHI, LTD) 8 December 2004 (2004-12-08) * paragraphs [0047], [0050] * ----- | 1-6,8-12 | |

TECHNICAL FIELDS SEARCHED (Int.Cl.7)
A61B

The present search report has been drawn up for all claims

| Place of search | Date of completion of the search | Examiner |
|---|---|---|
| Berlin | 4 March 2005 | Kronberger, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

3

EPO FORM 1503 03.82 (P04C01)

## ANNEX TO THE EUROPEAN SEARCH REPORT ON EUROPEAN PATENT APPLICATION NO. EP 04 00 1951

This annex lists the patent family members relating to the patent documents cited in the above-mentioned European search report.
The members are as contained in the European Patent Office EDP file on
The European Patent Office is in no way liable for these particulars which are merely given for the purpose of information.

04-03-2005

| Patent document cited in search report | | Publication date | Patent family member(s) | | | Publication date |
|---|---|---|---|---|---|---|
| US 5924996 | A | 20-07-1999 | DE | 4423663 | A1 | 11-01-1996 |
| | | | CA | 2194348 | A1 | 18-01-1996 |
| | | | CN | 1159151 | A | 10-09-1997 |
| | | | WO | 9601075 | A1 | 18-01-1996 |
| | | | EP | 0771168 | A1 | 07-05-1997 |
| | | | JP | 10503944 | T | 14-04-1998 |
| | | | KR | 271095 | B1 | 01-12-2000 |
| WO 0128414 | A | 26-04-2001 | WO | 0128414 | A2 | 26-04-2001 |
| EP 1484006 | A | 08-12-2004 | JP | 3566276 | B1 | 15-09-2004 |
| | | | JP | 2004329542 | A | 25-11-2004 |
| | | | EP | 1484006 | A1 | 08-12-2004 |
| | | | US | 2004225209 | A1 | 11-11-2004 |

EPO FORM P0459

For more details about this annex : see Official Journal of the European Patent Office, No. 12/82